# EUROPÄISCHE PATENTSCHRIFT

(11) **EP 4 208 113 B1**
(45) Veröffentlichungstag und Bekanntmachung des Hinweises auf die Patenterteilung: **28.01.2026**
(21) Anmeldenummer: 21787030.2
(22) Anmeldetag: 31.08.2021
(51) Int. Cl.: A61B 17/88, A61B 17/80

(54) **OPERATIONSZANGE ZUM HALTEN ZWEIER KNOCHENTEILE**
SURGICAL FORCEPS FOR HOLDING TWO BONE PARTS
PINCE CHIRURGICALE POUR LE MAINTIEN DE DEUX PARTIES OSSEUSES

(30) Priorität: 01.09.2020 DE 102020122829
(43) Veröffentlichungstag der Anmeldung: 12.07.2023
(73) Patentinhaber: Morina, Gazmend, 6430 Schwyz (CH)
(72) Erfinder: Morina, Gazmend, 6430 Schwyz (CH)
(74) Vertreter: Riebling, Peter
(86) Internationale Anmeldenummer: PCT/IB2021/057933
(87) Internationale Veröffentlichungsnummer: WO 2022/049479

(56) Entgegenhaltungen:
- WO-A1-2020/153615
- CN-A- 102 166 132
- GB-A- 2 506 373
- US-A- 2 583 896
- US-A1- 2018 168 707

## Beschreibung

Die vorliegende Erfindung betrifft eine Operationszange gemäß dem Oberbegriff des geltenden Anspruchs 1.

Eine solche Operationszange, auch Repositionszange oder Reponierungszange genannt, ist aus der Druckschrift CN 102 166 132 A oder aus der US 1 985 108 A bekannt.

WO2020153615A1 offenbart eine Zange zur internen Fixierung, die umfasst: ein erstes Element mit einem ersten Greifteil zum Greifen einer ersten Seitenfläche eines Knochens und einem ersten Griffteil, das sich vom ersten Greifteil erstreckt; und ein zweites Element mit einem zweiten Greifteil zum Greifen einer zweiten Seitenfläche des Knochens, die in einem vorbestimmten Abstand von der ersten Seitenfläche des Knochens beabstandet ist, und einem zweiten Griffteil, das sich vom zweiten Greifteil erstreckt.

US2018168707A1 beschreibt eine Klemmvorrichtung zur Knochenreduktion und internen Fixierung mit einer scherenähnlichen Struktur, die zwei Klemmelemente enthält, die schwenkbar miteinander verbunden sind und bogenförmige Elemente aufweisen, um Knochen und Fixierungsplatten während der Operation zu halten. Diese Vorrichtung wird speziell für die Behandlung von Trümmerbrüchen eingesetzt, indem sie den Knochen fest hält und Zugkraft zur Ausrichtung der Fraktur bietet, ohne dass das Gerät während der Operation entfernt werden muss.

Es ist Aufgabe der Erfindung, die bekannte Operationszange zu verbessern.

Die Aufgabe wird durch die Merkmale der unabhängigen Ansprüche gelöst.

Bevorzugte Weiterbildungen sind Gegenstand der abhängigen Ansprüche.

Gemäß einem Aspekt der Erfindung umfasst eine Operationszange zum Halten zweier Knochenteile eines gebrochenen Knochens an einer Knochenbruchstelle, ein sich in einer Radialrichtung erstreckendes erstes Zangenteil und ein sich in der Radialrichtung erstreckendes zweites Zangenteil, das in einer Drehrichtung um einen Drehpunkt über ein Schloss am ersten Zangenteil drehbar gehalten ist, wobei jedes Zangenteil in der Radialrichtung an einem Ende einen Zangenkopf mit einem Klemmabschnitt besitzt, so dass die Knochenteile zwischen den Zangenköpfen an den Klemmabschnitten einklemmbar sind. Erfindungsgemäß besitzt jedes Zangenteil zwischen den Klemmabschnitten und dem Schloss eine quer zur Radialrichtung und quer zur Drehrichtung verlaufende Schulter, an die eine Platte zur Osteosynthese der Knochenteile entgegen der Radialrichtung anlegbar ist.

Der Erfindung liegt die Überlegung zugrunde, dass für die Reponierung von Knochen herkömmlich mehrere Werkzeuge und/oder Hände notwendig sind. So sind neben dem eventuellen Vorbohren eines Knochens und dem Platzieren eines Kirschner-Drahtes einerseits die Knochenteile richtig zu positionieren und andererseits das Osteosynthesematerial richtig zu platzieren. Für beide Aufgaben kommen herkömmlich unterschiedliche Operationszangen zum Einsatz. Mit den eingangs erwähnten Operationszangen lassen sich zwar richtig positionierte Knochenteile gleichzeitig mit dem Osteosynthesematerial einklemmen und fixieren, allerdings sind zur richtigen Positionierung der Knochenteile und gleichzeitigen richtigen Anordnung des Osteosynthesematerials, also bis zum eigentlichen Klemmvorgang, immer noch mehrere Hände und/oder Werkzeuge notwendig.

Mit der angegebenen Operationszange wird vorgeschlagen, die Knochenteile nicht gleichzeitig mit dem Osteosynthesematerial sondern nacheinander zu klemmen. Hierzu kommt die Schulter zum Einsatz, die die Lage des Osteosynthesematerials in einer anderen Richtung sichert als die Klemmrichtung der Klemmabschnitte der Operationszange. Auf diese Weise lässt sich mit einem einzigen Werkzeug zunächst die Lage der Knochenteile richtig fixieren und im Anschluss die Lage des Osteosynthesematerials gleichzeitig mit dem Anlegen der Operationszange festlegen. Durch die Verwendung der angegebenen Operationszange kann daher die Anzahl der Operationswerkzeuge verringert werden.

In einer Weiterbildung umfasst die angegebene Operationszange in jedem Klemmabschnitt einen Abstandshalter in der Radialrichtung gesehen vor der Schulter, der eingerichtet ist, zwischen den Knochenteilen und der Schulter einen vorbestimmten Mindestabstand zu halten. Durch den Mindestabstand lässt sich zwar einerseits die Lage des Osteosynthesematerials an den fixierten Knochenteilen einstellen, allerdings mit einem gewissen mechanischen Spiel, welches es erlaubt, die relative Lage des Osteosynthesematerials an den gegeneinander fixierten Knochenteilen beliebig vor einem Verschrauben zu korrigieren.

In einer bevorzugten Weiterbildung der angegebenen Operationszange ist der Abstandshalter dadurch gebildet, dass die Klemmabschnitte in der Radialrichtung gesehen vor den Schultern bereichsweise aufeinander zu laufen. Auf diese Weise lässt sich der Abstandshalter einstückig mit den Klemmabschnitten ausbilden und somit kostengünstig in die Operationszange integrieren.

Erfindungsgemäß sind die Schultern als aufeinander zu gerichtete Vorsprünge zwischen der Klemmabschnitten und dem Schloss ausgebildet. Die Vorsprünge lassen sich beliebig entweder einstückig mit der Operationszange oder als extra Elemente an einer bereits existierenden Operationszange befestigen, so dass sich die Idee hinter der angegebenen Operationszange grundsätzlich auch an herkömmlichen Operationszangen durch Umbau realisieren lässt.

Erfindungsgemäß sind zum Bilden der Schultern der Zangenteile wenigstens zwei Vorsprünge nebeneinander angeordnet. Auf diese Weise lassen sich die einzelnen Vorsprünge kleiner ausbilden und so leichter beispielsweise bereits beim Urformen in die Operationszange miteinformen.

In einer noch anderen Weiterbildung der angegebenen Operationszange weist jeder Zangenkopf im Bereich seines Klemmabschnittes eine entgegen der Radialrichtung verlaufende Aussparung auf, die den Klemmabschnitt in einen ersten Gabelzinken und einen zweiten Gabelzinken trennt, und wobei die Gabelzinken eines Klemmbereiches quer zur Radialrichtung und quer zur Drehrichtung gesehen dünner sind, als die Gabelzinken des anderen Klemmbereiches.

In einer zusätzlichen Weiterbildung der angegebenen Operationszange sind die dünneren Gabelzinken auf einer Seite angeordnet, die der Einschraubseite einer Fixierschraube zur Osteosynthese der Knochenteile gegenüberliegt. Auf diese Weise lässt sich in einfacher Weise eine Schraube schräg durch die Aussparung und damit durch den zu reponierenden Knochen führen.

In einer weiteren Weiterbildung der angegebenen Operationszange sind die Gabelzinken in der Drehrichtung elastisch mit einem Elastizitätsmodul zwischen 30 GPa und 100 GPa ausgebildet. In diesem Elastizitätsbereich können die Gabelzinken Unebenheiten des Knochens in einfacher Weise ausgleichen und dennoch genügend Druckkraft aufbringen, um die Knochenteile sicher zu halten.

In einer noch weiteren Weiterbildung der angegebenen Operationszange sind die Gabelzinken in der Radialrichtung spitz zulaufend. Auf diese Weise lässt sich das Gewebe bei der Verwendung der angegebenen Operationszange besser schützen.

In einer zusätzlichen Weiterbildung der angegebenen Operationszange unterscheiden sich die Kontaktbereiche in ihrer Oberflächenrauheit, wodurch sich die angegebene Operationszange mit einem der beiden Klemmabschnitte gut positionieren lässt, während der andere der beiden Klemmabschnitte beim Klemmen eine Lagestabilität herstellt.

Die oben beschriebenen Eigenschaften, Merkmale und Vorteile dieser Erfindung sowie die Art und Weise, wie diese erreicht werden, werden verständlicher im Zusammenhang mit der folgenden Beschreibung der Ausführungsbeispiele, die im Zusammenhang mit der Zeichnung näher erläutert werden. Es zeigen:
Fig. 1 eine schematische Ansicht einer Operationszange in Form einer Reponierungszange,
Fig. 2 die Reponierungszange aus Fig. 1 in einem Anwendungszustand,
Fig. 3 eine schematische Ansicht von Klemmabschnitten der Reponierungszange aus Fig. 1,
Fig. 4 einen Knochen mit einem Schrägdrehbruch,
Fig. 5 die Reponierungszange aus Fig. 1 am Knochen der Fig. 4 aus einer ersten Perspektive,
Fig. 6 die Reponierungszange aus Fig. 1 am Knochen der Fig. 4 aus einer zweiten Perspektive,
Fig. 7 einen Knochen mit einem Querbruch, und
Fig. 8 die Reponierungszange aus Fig. 1 am Knochen der Fig. 7.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

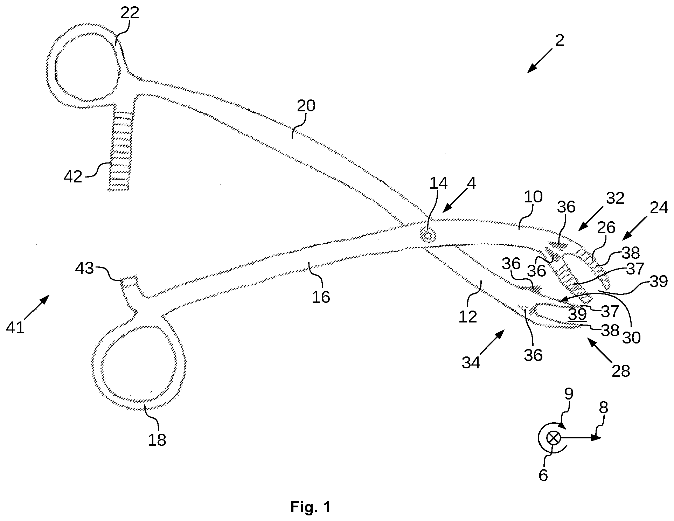

Es wird auf Fig. 1 Bezug genommen, die eine schematische Ansicht einer Operationszange, nachstehend Reponierungszange 2 genannt, zeigt.

Die Reponierungszange 2 wird nachstehend in einem Raum beschrieben, der aus Sicht eines noch zu beschreibenden Drehpunktes 4 von einer aus der Bildebene herauszeigenden Axialrichtung 6, einer quer zur Axialrichtung 6 in der Bildebene verlaufenden Radialrichtung 8 und einer um die Axialrichtung 6 herumlaufenden Drehrichtung 9 aufgespannt wird.

Die Reponierungszange 2 umfasst ein sich in der Radialrichtung 8 erstreckendes erstes Zangenteil 10 und ein sich in der Radialrichtung 8 erstreckendes zweites Zangenteil 12, das in der Drehrichtung 9 um den Drehpunkt 4 über ein Schloss 14 am ersten Zangenteil 10 drehbar gehalten ist. An das erste Zangenteil 10 schließt sich eine erste Branche 16 mit einem ersten Griffelement 18 an, während sich an das zweite Zangenteil 12 eine zweite Branche 20 mit einem zweiten Griffelement 22 anschließt. Die Reponierungszange 2 ist in der vorliegenden Ausführung als Gelenkzange ausgebildet, weshalb sich die Branchen 16, 20 mit den Griffelementen 18, 22 an die Zangenteile 10, 12 auf einer dem Schloss 14 gegenüberliegenden Seite anschließen. Wäre die Reponierungszange 2 als Bügelzange ausgeführt, lägen die Branchen 16, 20 mit den Griffelementen 18, 22 zwischen den Zangenteilen 10, 12 und dem Schloss 14. Auch wenn die Erfindung am Beispiel einer Gelenkzange gezeigt wird, ist sie nicht auf eine solche eingeschränkt.

Das erste Zangenteil 10 besitzt in der Radialrichtung 8, auch Längsrichtung genannt, an einem dem Schloss 14 gegenüberliegenden Ende einen ersten Zangenkopf 24 mit einem ersten Klemmabschnitt 26, während das zweite Zangenteil 12 in der Radialrichtung 8 an einem dem Schloss 14 gegenüberliegenden Ende einen zweiten Zangenkopf 28 mit einem in der Perspektive der Fig. 1 nicht zu sehenden zweiten Klemmabschnitt 30 besitzt. Die beiden Klemmabschnitte 26, 30 sind in der Drehrichtung 9 aufeinander zu gerichtet. Auf diese Weise lässt sich ein Knochen zwischen den Zangenköpfen 24, 28 an den Klemmabschnitten 26, 30 einklemmen, worauf an späterer Stelle näher eingegangen wird.

Erfindungsgemäß umfasst das erste Zangenteil 10 zwischen seinem Klemmabschnitt 26 und dem Schloss 14 eine in der Axialrichtung 6 verlaufende erste Schulter 32, während das erste Zangenteil 12 zwischen seinem Klemmabschnitt 30 und dem Schloss 14 eine in der Axialrichtung 6 verlaufende zweite Schulter 34 umfasst. An die Schultern 32, 34 lässt sich eine Platte zur Osteosynthese entgegen der Radialrichtung anlegen und so gemeinsam mit der Reponierungszange 2 in der Radialrichtung 2 gegen einen Knochen drücken. Hierauf wird an späterer Stelle näher eingegangen.

Die Schultern 32, 34 sind Erfindungsgemäß aus je zwei Vorsprüngen 36 gebildet. Dabei ist einer der Vorsprünge 36 in der Perspektive der Fig. 1 nicht zu sehen und daher nur gestrichelt angedeutet.

Jeder Zangenkopf 24, 28 weist in seinem Klemmabschnitt 26, 30 eine entgegen der Radialrichtung 8 verlaufende Aussparung 37 auf, die den jeweiligen Klemmabschnitt 26, 30 in einen ersten Gabelzinken 38 und einen zweiten Gabelzinken 39 trennt.

Zwischen den Branchen 16, 20 und den Griffelementen 18, 22 ist ein Verriegelungsmechanismus 41 angeordnet. Der Verriegelungsmechanismus 41 umfasst eine Zahnstange 42, die sich entgegen der Drehrichtung 9 von der zweiten Branche 20 weg erstreckt, während sich ein in die Zahnstange 42 eingreifender Verriegelungszahn 43 in der Drehrichtung 9 von der ersten Branche 16 weg erstreckt. Auf der Zahnstange 42 sind zwischen sieben und zwölf Zähne ausgebildet, in die der Verriegelungszahn 43 in der Drehrichtung 9 formschlüssig eingreifen und die Zangenteile 10, 12 in vorbestimmten Winkelpositionen zueinander fixiert positionieren kann.

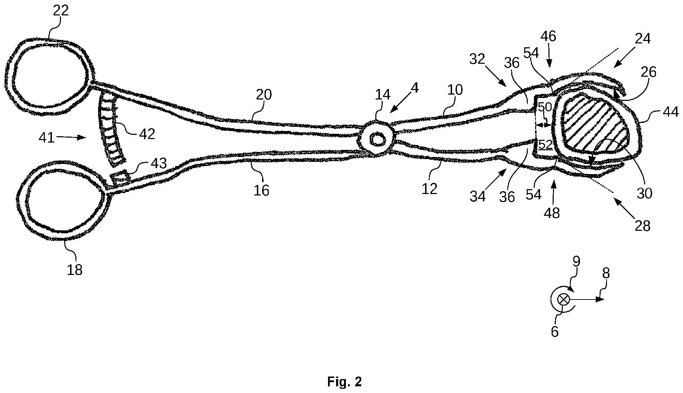

Auf weitere Details der Reponierungszange 2 wird nachstehend anhand von Fig. 2 eingegangen, die die Reponierungszange 2 aus Fig. 1 in einem Anwendungszustand zeigt, in dem zwischen den beiden Klemmabschnitten 26, 30 ein Knochen 44 eingeklemmt ist.

Die Reponierungszange 2 umfasst am ersten Zangenteil 10 zwischen dem ersten Klemmabschnitt 26 und der ersten Schulter 32 einen ersten Abstandshalter 46, während sie am zweiten Zangenteil 12 zwischen dem zweiten Klemmabschnitt 30 und der zweiten Schulter 34 einen zweiten Abstandshalter 48 umfasst. Die beiden Abstandshalter 46, 48 sind eingerichtet, den Knochen 44 in einem Mindestabstand 50 vor den Schultern 32, 34 zu halten. Damit schaffen die Abstandshalter 46, 48 einen Raum 52, in dem die oben beschriebene Platte zur Osteosynthese nicht nur aufgenommen sondern auch nach Einklemmen des Knochens 44 immer noch richtig positioniert werden kann.

In der vorliegenden Ausführung sind die Abstandshalter 46, 48 Kanten 54, die dadurch gebildet sind, dass die Klemmabschnitte 26, 30 in der Radialrichtung 8 gesehen von den Schultern 32, 34 auseinander laufen, so dass die sich in der Axialrichtung 6 erstreckenden Kante 54 entstehen. Das Auseinanderlaufen ist in Fig. 2 durch gestrichelte Linien angedeutet.

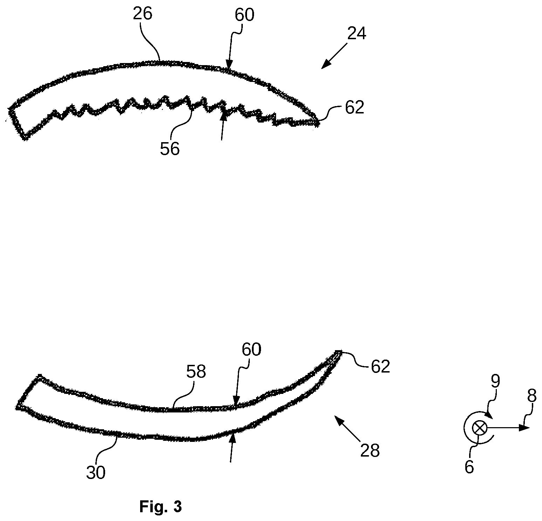

Bevor auf die Verwendung der Reponierungszange 2 näher eingegangen wird, soll nachstehend noch anhand Fig. 3 auf die Klemmabschnitte 26, 30 der Reponierungszange 2 aus Fig. 1 eingegangen werden.

Der erste Klemmabschnitt 26 weist in der vorliegenden Ausführung an seiner zum Knochen 44 anliegenden Seite Rändel 56 auf, die ein Verrutschen des Knochens 44 verhindern, wenn der Knochen 44 zwischen den Klemmabschnitten 26, 30 eingeklemmt ist. Demgegenüber ist die Oberfläche 58 des zweiten Klemmabschnitts 30, die am Knochen 44 anliegt, glatt ausgeführt und erlaubt vor dem Einklemmen des Knochen 44 eine exakte Positionierung in der Radialrichtung. Allerdings können grundsätzlich beide Klemmabschnitte 24, 28 mit den Rändeln 56 oder auch beide Klemmabschnitte 24, 28 glatt ausgeführt sein.

Die beiden Klemmabschnitte 24, 26 sind flach, das heißt mit einer geringen Dicke 60 in der Drehrichtung 9 ausgebildet. Zudem sind beide Klemmabschnitte 24 an ihren vom Schloss 14 weg gerichteten Enden mit je einer scharfen Spitze 62 ausgebildet. In dieser Ausführung kann die Reponierungszange 2 gewebeschonend am Knochen 44 angreifen.

Nachstehend wird die Verwendung der Reponierungszange 2 anhand zweier Beispiele näher erläutert.

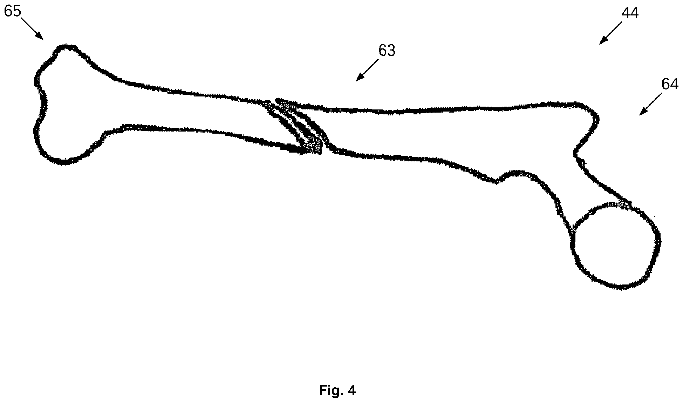

Zunächst wird die Verwendung der Reponierungszange 2 zur Operation eines Schrägdrehbruches erläutert. Hierzu wird auf Fig. 4 Bezug genommen, die den Knochen 44 mit einem Schrägdrehbruch 63 zeigt.

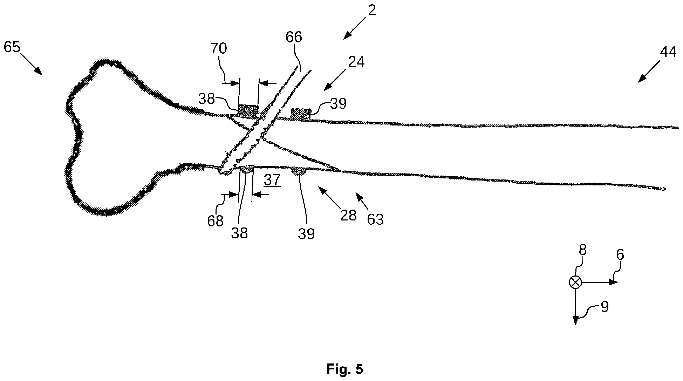

Bei dem Knochen 44 der Fig. 5 handelt es sich um einen Oberschenkelknochen mit einem proximalen Ende 64, welches zum Oberkörper hin gerichtet ist und einem distalen Ende 65, welches zum Fuß gerichtet ist.

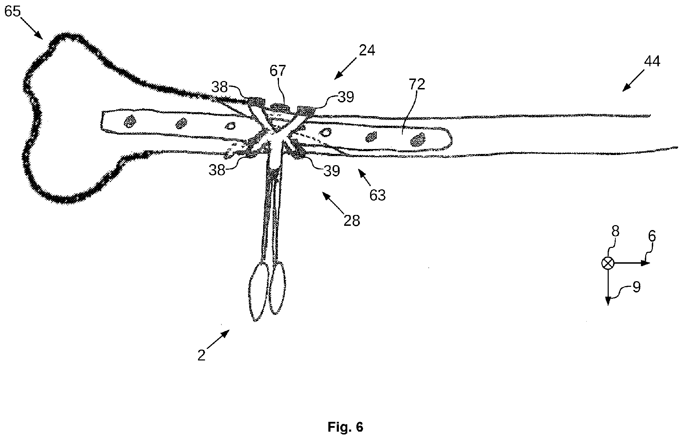

Die Operation dieses Schrägdrehbruches 63 mit der Reponierungszange 2 soll nachstehend anhand der Fig. 5 und 6 näher erläutert werden.

Sind die beiden nicht weiter referenzierten Knochenteile des Knochens 44 gegeneinander positioniert, wird der Knochen 44, wie in Fig. 5 gezeigt, zwischen den Zangenköpfen 24, 28 der Reponierungszange 2 eingeklemmt.

Danach wird zur Vorbereitung eines Einschraubens einer in Fig. 6 gezeigten Zugschraube 67 mit einem Bohrer 66 eine Bohrung gebohrt. Der Bohrer 66 wird zwischen den beiden Gabelzinken 38, 39 des ersten Zangenkopfes 24 angesetzt und in der Aussparung 37 möglichst rechtwinklig zum Schrägdrehbruch 63 durch den Knochen 44 getrieben, so dass der Bohrer 66 außerhalb des zweiten Zangenkopfes 28 aus dem Knochen 44 wieder austritt.

Um dies möglichst zuverlässig zu gewährleisten, sind die Gabelzinken 38, 39 des zweiten Zangenkopfes 28 in der Axialrichtung 6 gesehen mit einer Breite 68 ausgebildet, die kleiner ist, als eine Breite 70 der Gabelzinken 38, 39 des ersten Zangenkopfes 24.

Im Anschluss an die Bohrung kann dann die Zugschraube 67 eingeschraubt werden. Parallel dazu kann in den oben erwähnten Raum 52 die Platte zur Osteosynthese eingeschoben werden, die in Fig. 5 mit dem Bezugszeichen 72 versehen ist. Nachdem die Platte 72 positioniert und mit nicht weiter referenzierten Schrauben fixiert ist, kann die Reponierungszange 2 wieder gelöst werden. Weitere Werkzeuge neben der Reponierungszange 2 sind zur Reponierung des Knochens 44 und zur Positionierung der Platte zur Osteosynthese während der Operation des Schrägdrehbruches 63 nicht notwendig. Gegebenenfalls können für andere Tätigkeiten weitere Werkzeuge notwendig werden, wie beispielsweise ein Werkzeug zum Halten der Weichteile.

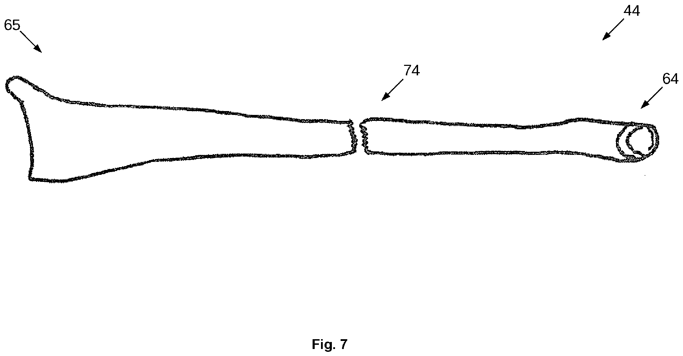

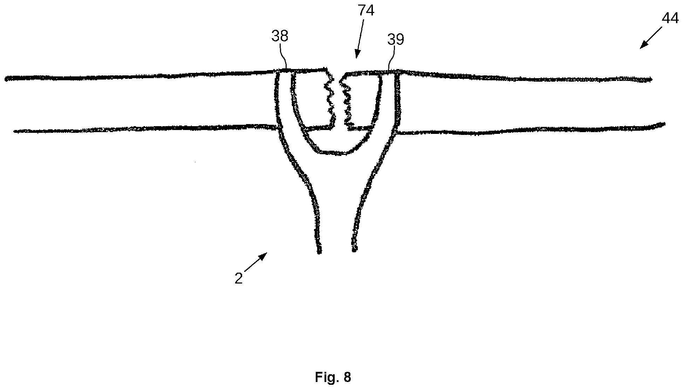

In gleicher Weise kann mit der Reponierungszange 2 auch ein Querbruch 74 operiert werden, was in den Fig. 7 und 8 an einem Unterarmknochen angedeutet ist.

## Patentansprüche

1. Operationszange (2) zum Halten zweier Knochenteile eines gebrochenen Knochens (44) an einer Knochenbruchstelle (63, 74), umfassend ein sich in einer Radialrichtung (8) erstreckendes erstes Zangenteil (10) und ein sich in der Radialrichtung (8) erstreckendes zweites Zangenteil (12), das in einer Drehrichtung (9) um einen Drehpunkt (6) über ein Schloss (14) am ersten Zangenteil (10) drehbar gehalten ist, wobei jedes Zangenteil (10, 12) in der Radialrichtung (8) an einem Ende einen Zangenkopf (24, 28) mit einem Klemmabschnitt (26, 30) besitzt, so dass die Knochenteile zwischen den Zangenköpfen (24, 28) an den Klemmabschnitten (26, 30) einklemmbar sind, wobei jedes Zangenteil (10, 12) zwischen dem Klemmabschnitt (26, 30) und dem Schloss (14) eine quer zur Radialrichtung (8) und quer zur Drehrichtung (9) verlaufende Schulter (32, 34) besitzt, an die eine Platte (72) zur Osteosynthese der Knochenteile entgegen der Radialrichtung (8) anlegbar ist, wobei die Schultern (32, 34) als aufeinander zu gerichtete Vorsprünge (36) zwischen den Klemmabschnitten (26, 30) und dem Schloss (14) ausgebildet und zum Bilden der Schultern (32, 34) der Zangenteile (10, 12) wenigstens zwei Vorsprünge (36) nebeneinander angeordnet sind.

2. Operationszange (2) nach Anspruch 1, umfassend einen Abstandshalter (46, 48) in wenigstens einem Klemmabschnitt (26, 30) in der Radialrichtung (8) gesehen vor der Schulter (32, 34), der eingerichtet ist, zwischen den Knochenteilen und der Schulter (32, 34) einen vorbestimmten Mindestabstand (50) zu halten.

3. Operationszange (2) nach Anspruch 2, wobei der Abstandshalter (46, 48) dadurch gebildet ist, dass der wenigstens eine Klemmabschnitt (26, 30) in der Radialrichtung (8) gesehen vor den Schultern (32, 34) bereichsweise auseinander laufen.

4. Operationszange (2) nach einem der vorstehenden Ansprüche, wobei jeder Zangenkopf (24, 28) im Bereich seines Klemmabschnittes (26, 30) eine entgegen der Radialrichtung (8) verlaufende Aussparung (37) aufweist, die den Klemmabschnitt (26, 30) in einen ersten Gabelzinken (38) und einen zweiten Gabelzinken (39) trennt, und wobei die Gabelzinken (38, 39) eines Klemmabschnittes (26, 30) quer zur Radialrichtung (8) und quer zur Drehrichtung (9) gesehen dünner sind als die Gabelzinken (38, 39) des anderen Klemmabschnittes (26, 30).

5. Operationszange (2) nach Anspruch 4, wobei die dünneren Gabelzinken (38, 39) auf einer Seite angeordnet sind, die der Einschraubseite einer Fixierschraube (67) zur Osteosynthese der Knochenteile gegenüberliegt.

6. Operationszange (2) nach Anspruch 4 oder 5, wobei die Gabelzinken (38, 39) in der Drehrichtung (9) elastisch mit einem Elastizitätsmodul zwischen 30 GPa und 100 GPa ausgebildet sind.

7. Operationszange (2) nach einem der Ansprüche 4 bis 6, wobei die Gabelzinken (38, 39) in der Radialrichtung (8) spitz zulaufend sind.

8. Operationszange (2) nach einem der vorstehenden Ansprüche, wobei sich die Klemmabschnitte (26, 30) in ihrer Oberflächenrauheit unterscheiden.

## Claims

1. Surgical forceps (2) for holding two bone parts of a broken bone (44) at a bone fracture site (63, 74), comprising a first forceps part (10) extending in a radial direction (8) and a second forceps part (12) extending in the radial direction (8), which is held to be rotatable in a rotational direction (9) about a pivot point (6) via a lock (14) on the first forceps part (10), wherein each forceps part (10, 12) has a forceps head (24, 28) with a clamping section (26, 30) at one end in the radial direction (8), so that the bone parts can be clamped between the forceps heads (24, 28) at the clamping sections (26, 30), each forceps part (10, 12) having a shoulder (32, 34) that extends transversely to the radial direction (8) and transversely to the rotational direction (9) between the clamping section (26, 30) and the lock (14), to which shoulder a plate (72) for osteosynthesis of the bone parts can be applied against the radial direction (8), wherein the shoulders (32, 34) are designed as projections (36) pointing towards each other between the clamping sections (26, 30) and the lock (14), and at least two projections (36) are arranged next to each other to form the shoulders (32, 34) of the forceps parts (10, 12).

2. The surgical forceps (2) according to claim 1,
comprising a spacer (46, 48) in at least one clamping section (26, 30) in front of the shoulder (32, 34) as seen in the radial direction (8), which spacer is designed to maintain a predetermined minimum distance (50) between the bone parts and the shoulder (32, 34).

3. The surgical forceps (2) according to claim 2, wherein the spacer (46, 48) is formed by the at least one clamping section (26, 30) diverging in areas in front of the shoulders (32, 34) as seen in the radial direction (8).

4. The surgical forceps (2) according to any one of the preceding claims, wherein each forceps head (24, 28) has a recess (37) in the area of its clamping section (26, 30) which extends in the opposite direction to the radial direction (8) and separates the clamping section (26, 30) into a first fork prong (38) and a second fork prong (39), and wherein the fork prongs (38, 39) of one clamping section (26, 30) are thinner than the fork prongs (38, 39) of the other clamping section (26, 30) as seen transversely to the radial direction (8) and transversely to the rotational direction (9).

5. The surgical forceps (2) according to claim 4, wherein the thinner fork prongs (38, 39) are arranged on a side opposite the screw-in side of a fixation screw (67) for osteosynthesis of the bone parts.

6. The surgical forceps (2) according to claim 4 or 5,
wherein the fork prongs (38, 39) are designed to be elastic in the rotational direction (9) with a modulus of elasticity between 30 GPa and 100 GPa.

7. The surgical forceps (2) according to any one of claims 4 to 6, wherein the fork prongs (38, 39) taper in the radial direction (8).

8. The surgical forceps (2) according to any one of the preceding claims, wherein the clamping sections (26, 30) differ in their surface roughness.

## Revendications

1. Pince chirurgicale (2) pour maintenir deux parties osseuses d'un os fracturé (44) en un site de fracture osseuse (63, 74), comprenant une première partie de pince (10) s'étendant dans une direction radiale (8) et une seconde partie de pince (12) s'étendant dans la direction radiale (8), qui est maintenue avec possibilité de rotation dans une direction de rotation (9) autour d'un point de rotation (6) par l'intermédiaire d'un verrou (14) sur la première partie de pince (10), dans laquelle chaque partie de pince (10, 12) possède, dans la direction radiale (8), à une extrémité, une tête de pince (24, 28) comportant une section de serrage (26, 30), de telle sorte que les parties osseuses puissent être enserrées entre les têtes de pince (24, 28) en des sections de serrage (26, 30), dans laquelle chaque partie de pince (10, 12) possède, entre la section de serrage (26, 30) et le verrou (14), un épaulement (32, 34) se prolongeant transversalement à la direction radiale (8) et transversalement à la direction de rotation (9), sur lequel une plaque (72) peut être appliquée pour l'ostéosynthèse des parties osseuses à l'opposé de la direction radiale (8), dans laquelle les épaulements (32, 34) sont réalisés en tant que saillies (36) orientées l'une vers l'autre entre les sections de serrage (26, 30) et le verrou (14), et au moins deux saillies (36) sont agencées côte à côte pour former les épaulements (32, 34) des parties de pince (10, 12).

2. Pince chirurgicale (2) selon la revendication 1, comprenant un écarteur (46, 48) dans au moins une section de serrage (26, 30), devant l'épaulement (32, 34) vu dans la direction radiale (8), qui est destiné à maintenir une distance minimale (50) prédéterminée entre les parties osseuses et l'épaulement (32, 34).

3. Pince chirurgicale (2) selon la revendication 2, dans laquelle l'écarteur (46, 48) est formé de telle sorte que l'au moins une section de serrage (26, 30) s'écarte par régions, devant les épaulements (32, 34), vue dans la direction radiale (8).

4. Pince chirurgicale (2) selon l'une des revendications précédentes, dans laquelle chaque tête de pince (24, 28) présente dans la zone de sa section de serrage (26, 30) un évidement (37) se prolongeant à l'opposé de la direction radiale (8), qui sépare la section de serrage (26, 30) en une première dent de fourche (38) et une seconde dent de fourche (39), et dans laquelle les dents de fourche (38, 39) d'une section de serrage (26, 30), vues transversalement à la direction radiale (8) et transversalement à la direction de rotation (9), sont plus minces que les dents de fourche (38, 39) de l'autre section de serrage (26, 30).

5. Pince chirurgicale (2) selon la revendication 4, dans laquelle les dents de fourche (38, 39) plus fines sont agencées d'un côté opposé au côté de vis de la vis de fixation (67) pour l'ostéosynthèse des parties osseuses.

6. Pince chirurgicale (2) selon la revendication 4 ou 5, dans laquelle les dents de fourche (38, 39) sont réalisées de manière élastique dans la direction de rotation (9) avec un module d'élasticité compris entre 30 GPa et 100 GPa.

7. Pince chirurgicale (2) selon l'une des revendications 4 à 6, dans laquelle les dents de fourche (38, 39) sont pointues dans la direction radiale (8).

8. Pince chirurgicale (2) selon l'une des revendications précédentes, dans laquelle les sections de serrage (26, 30) se distinguent par leur rugosité de surface.
